# EUROPEAN PATENT SPECIFICATION

(11) **EP 2 449 129 B1**
(45) Date of publication and mention of the grant of the patent: **16.04.2014**
(21) Application number: 10794686.5
(22) Date of filing: 30.06.2010
(51) Int. Cl.: G01N 33/84, G01N 35/08, C12Q 1/00

(54) **METHODS OF DETERMINING THE PRESENCE AND/OR CONCENTRATION OF AN ANALYTE IN A SAMPLE**
VERFAHREN ZUR BESTIMMUNG DES VORHANDENSEINS UND/ODER DER KONZENTRATION EINES ANALYTS IN EINER PROBE
PROCÉDÉS POUR DÉTERMINER LA PRÉSENCE ET/OU LA CONCENTRATION D UN ANALYTE DANS UN ÉCHANTILLON

(30) Priority: 01.07.2009 US 222285 P
(43) Date of publication of application: 09.05.2012
(73) Proprietor: Board of Regents, The University of Texas System, Austin, TX 78701 (US); Henry Ford Health System, Detroit, MI 48202 (US)
(72) Inventor: ANSLYN, Eric, V., Austin, TX 78723 (US); YANG, Youjun, Austin, TX 78723 (US); SZAMOSFALVI, Balazs, Bloomfield Hills, MI 48301 (US); YEE, Jerry, Beverly Hills, MI 48025 (US); FRINAK, Stanley, Farmington Hills, MI 48334 (US)
(74) Representative: Potter Clarkson LLP
(86) International application number: PCT/US2010/040543
(87) International publication number: WO 2011/002850

(56) References cited:
- US-A1- 2007 259 450
- US-A1- 2008 015 487
- US-A1- 2008 219 891
- US-B2- 6 801 316
- US-B2- 7 302 349
- YOUJUN YANG ET AL: "Development of an online citrate/Ca2+ sensing system for dialysis", THE ANALYST, vol. 136, no. 2, 20 October 2010 (2010-10-20), page 317, XP55021610, ISSN: 0003-2654, DOI: 10.1039/c0an00647e
- JAVEED SHAIKH MOHAMMED ET AL: "Microfluidic device for multimodal characterization of pancreatic islets", LAB ON A CHIP, vol. 9, no. 1, 1 January 2009 (2009-01-01) , page 97, XP55021721, ISSN: 1473-0197, DOI: 10.1039/b809590f
- VIMAL CHADHA ET AL: "Citrate clearance in children receiving continuous venovenous renal replacement therapy", PEDIATRIC NEPHROLOGY, vol. 17, no. 10, 1 October 2002 (2002-10-01), pages 819-824, XP55021741, ISSN: 0931-041X, DOI: 10.1007/s00467-002-0963-6
- KOZIK-JAROMIN JUSTYNA ET AL: "Citrate pharmacokinetics and calcium levels during high-flux dialysis with regional citrate anticoagulation.", NEPHROLOGY, DIALYSIS, TRANSPLANTATION : OFFICIAL PUBLICATION OF THE EUROPEAN DIALYSIS AND TRANSPLANT ASSOCIATION - EUROPEAN RENAL ASSOCIATION JUL 2009 LNKD- PUBMED:19196824, vol. 24, no. 7, 5 February 2009 (2009-02-05), pages 2244-2251, XP002671564, ISSN: 1460-2385

## Description

### BACKGROUND

Continuous renal replacement therapy (CRRT) is a form of extracorporeal blood treatment (EBT) that is performed in the intensive care unit (ICU) for patients with acute renal failure (ARF) or end-stage renal disease (ESRD), who are often hemodynamically unstable with multiple co-morbidities. In a specific form of CRRT, continuous veno-venous hemofiltration (CVVH), blood is pumped through a hemofilter and uremic toxin-laden plasma ultrafiltrate is discarded at a rate of 1-10 liters per hour (convective removal of solutes). An equal amount of sterile crystalloid solution (replacement fluid, CRRT fluid) with physiological electrolyte and base concentrations is simultaneously infused into the blood circuit either before the hemofilter (pre-dilution) or after the hemofilter (post-dilution) to avoid volume depletion and hemodynamic collapse.

From a theoretical and physiological point of view, when run continuously for 24 hours per day, CVVH is the closest of all available renal replacement therapy (RRT) modalities today to replicate the function of the native kidneys and the preferred treatment modality for critically ill patients with renal failure. Nevertheless, 90% of RRT in the ICU is performed as intermittent hemodialysis (IHD), sustained low efficiency dialysis (SLED), or sometimes as continuous veno-venous hemo-diafiltration (CVVHDF). Common to all of these latter methods of RRT is that the removal of most solutes is predominantly by the process of diffusion from blood plasma through the membrane of the hemofilter into the dialysis fluid. Diffusion is less efficient in the removal of larger solutes and also provides less predictable small solute movement than convection and therefore, from a theoretical standpoint, CVVH is a superior method of RRT.

The most important reason for the limited use of CVVH in the ICU is that anticoagulation is mandatory to prevent clotting of the extracorporeal circuit in 24-hour treatments. Systemic anticoagulation has an unacceptable rate of major bleeding complications in critically ill patients and cannot be done safely. Similarly, extracorporeal blood treatments including plasmapheresis, plasma adsorption on specialized columns, blood banking procedures, lipid apheresis systems, plasma adsorption-based endotoxin removal, treatment with a bioartificial kidney device that contains live renal tubular cells, or with a liver replacement therapy circuit also require powerful anticoagulation..

Regional citrate anticoagulation (RCA) has emerged as a possible solution to the clinical problem of circuit clotting without inducing any systemic bleeding tendency. Citrate, a trivalent anion, is present in the human plasma as an intermediate of metabolism. This ion chelates ionized calcium in the plasma resulting in a single negative Ca-citrate complex and decreased free ionized calcium levels. Since the coagulation cascade requires free ionized calcium for optimal function, blood clotting in the extracorporeal blood circuit (EBC) can be completely prevented by an infusion of citrate into the arterial (incoming) limb of the EBC. When the blood is passed through the extracorporeal processing unit, the anticoagulant effect can be fully reversed by the local infusion of free ionized calcium into the venous (return) limb of the EBC. Therefore, theoretically, regional citrate anticoagulation can be both very powerful and fully reversible without systemic (intra-patient) bleeding tendencies.

Regional citrate anticoagulation has been performed for more than 20 years. Due to the lack of a simple and efficient protocol for the analysis of the critical composition of ultrafiltrate or blood, however, a number of complications associated with the practice of RCA occur. The following complications are well documented: hypernatremia; metabolic alkalosis; metabolic acidosis, hypocalcemia 1 (due to net calcium loss from the patient), hypocalcemia 2 (due to systemic citrate accumulation), rebound hypercalcemia (due to release of calcium from citrate after CVVH is stopped), hypophosphatemia, fluctuating levels of anticoagulation, nursing and physician errors, ionized hypomagnesemia, declining filter performance, trace metal depletion, etc. All these may be solved if real time monitoring of analytes, specifically citrate and ionized calcium is made possible.

Additionally, using the above CVVH system, the patient's systemic plasma citrate level can fluctuate in the 0-3 mmol/L range depending on the body metabolism of citrate. Since an accumulation of systemic citrate to 3 mM could result in significant systemic ionized hypocalcemia unless the calcium infusion is increased to proportionally increase the plasma total calcium level, it is necessary to monitor the systemic citrate and total calcium levels.

Laboratory testing of citrate and ionized calcium is not available in the routine clinical ICU setting. Marked changes in citrate and calcium levels can also develop in 2-3 hours during CVVH, too quickly for routine plasma chemistry monitoring every 6 hours to detect such problems in a timely manner before they have adverse clinical sequelae. The effluent fluid contains a wealth of information on the patient's plasma solute composition. This fluid is a clear crystalloid with a small amount of albumin, small peptides, and cytokines also present. The transparency and minimal viscosity of the effluent fluid provide for an ideal environment for an optical- and/or chemical sensor array. However, in current clinical practice, it is discarded without any further analysis.

Furthermore, reduced Mg(II) concentration in blood, known as hypomagnesemia, may lead to weakness, muscle cramps, cardiac arrhythmia, increased irritability of the nervous system with tremors, athetosis, jerking, nystagmus and an extensor plantar reflex. In addition, there may be confusion, disorientation, hallucinations, depression, epileptic fits, hypertension, tachycardia and tetany. However, due to the lack of convenient and reliable clinical monitoring protocol of magnesium, a 2.5:1 molar ratio between total plasma calcium and total plasma magnesium is usually maintained by using a high-Mg commercial replacement fluid. Phosphate losses can also be very large and can quickly lead to severe hypophosphatemia with high daily clearance goals during CVVH unless phosphate is added to the CRRT replacement fluid.

A need exists to develop an effective, real time method to measure the level of analytes, such as citrate, ionized calcium, magnesium, phosphate, etc., in the clinical setting. Such a method may take advantage of the effluent fluid that is currently discarded.

### SUMMARY

The present disclosure relates generally to methods of determining the presence and/or concentration level of an analyte in a sample as set out in the claims. More particularly, in some embodiments, the present disclosure relates to methods of measuring the concentration of citrate, ionized calcium, magnesium and/or phosphate in a sample.

In one embodiment, the present disclosure provides a method for continuous, real-time monitoring of concentration levels of an analyte in a biological fluid comprising an extracorporeal blood circuit effluent fluid produced on a hemofilter or a dialyzer device with hemofiltration or dialysis or any combination of these two processes, the method comprising: providing an analyte; providing an analyte receptor and an indicator, wherein at least a portion of the analyte receptor and the indicator form a receptor/indicator complex; contacting the receptor/indicator complex with the analyte; and allowing the analyte to interact with the receptor/indicator complex so as to generate a detectable signal; and detecting the signal by using a Flow Injection Analysis instrument.

In another embodiment, the present disclosure provides a system for continuous, real-time monitoring of concentration levels of an analyte in a biological fluid comprising an extracorporeal blood circuit effluent fluid produced on a hemofilter or a dialyzer device with hemofiltration or dialysis or any combination of these two processes, the system comprising: a receptor/indicator complex comprising an analyte receptor and an indicator; and an analyte, wherein the analyte will displace the indicator in the receptor/indicator complex; and wherein the displaced indicator will generate a detectable signal; and a Flow Injection Analysis instrument.

The features and advantages of the present disclosure will be readily apparent to those skilled in the art upon a reading of the description of the embodiments that follows.

### DRAWINGS

A more complete understanding of this disclosure may be acquired by referring to the following description taken in combination with the accompanying figures in which:
FIGURE 1 is an image depicting a mechanism of analyte sensing via an indicator displacement assay, according to one embodiment.
FIGURE 2 is an image depicting a mechanism of analyte sensing via an analyte (Ca²⁺) binding to a receptor/indicator complex (Fura-2), according to one embodiment.
FIGURES 3A and 3B depict the structure of representative citrate receptors, according to one embodiment.
FIGURE 4 depicts several representative Ca²⁺ receptors, according to one embodiment.
FIGURE 5 depicts several representative Mg²⁺ receptors, according to one embodiment.
FIGURE 6 depicts the synthesis scheme of Mg²⁺ receptors 2 and 3 (Figure 5) from known compounds, according to one embodiment.
FIGURE 7 depicts representative Mg²⁺ receptors, according to one embodiment.
FIGURE 8 depicts representative phosphate receptors based on H-bpmp, according to one embodiment
FIGURE 9 is an image depicting a mechanism of analyte sensing via indicator displacement assay using H-bpmp, according to one embodiment.
FIGURE 10 depicts changes of the solution UV-Vis spectra containing a H-bpmp receptor and a pyrocatechol violet indicator upon the addition of a phosphate analyte, according to one embodiment.
FIGURE 11 depicts the structure of representative indicators, according to one embodiment.
FIGURES 12A and 12B depict sample calibration curves for citrate (11A) and Ca²⁺(11B), according to one embodiment.
FIGURE 13 depicts the working principle of a Flow-Injection-Analysis ("FIA") instrument, according to one embodiment.
FIGURE 14 is a schematic representation of a FIA instrument, according to one embodiment.
FIGURE 15 depicts the proposed binding modes of Receptor 2 with alizarin complexone and citrate.
FIGURE 16A depicts changes of the solution UV-Vis spectra containing both Receptor 2, and Alizarin Complexone upon addition of citrate, according to one embodiment. Arrows indicate the spectral changes upon increasing citrate concentration.
FIGURE 16B depicts the extrapolated calibration curve of citrate concentration by monitoring the absorbance at 540 nm, according to one embodiment.
FIGURE 17A depicts changes of the solution UV-Vis spectra containing Fura-2 upon addition of Ca²⁺, according to one embodiment. Arrows indicates the spectral changes upon increasing the Ca²⁺ concentration.
FIGURE 17B depicts extrapolated calibration curve of the Ca²⁺ concentration by monitoring the solution absorbance at 373 nm, according to one embodiment.

While the present disclosure is susceptible to various modifications and alternative forms, specific example embodiments have been shown in the figures and are herein described in more detail. It should be understood, however, that the description of specific example embodiments is not intended to limit the disclosure to the particular forms disclosed, but on the contrary, this disclosure is to cover all modifications and equivalents as defined by the appended claims.

### DESCRIPTION

The present disclosure relates generally to methods of determining the presence and/or concentration level of an analyte in a sample as set out in the claims. More particularly, in some embodiments, the present disclosure relates to methods of determining the presence and/or concentration level of citrate, ionized calcium, magnesium and/or phosphate in a sample.

In one embodiment, the present disclosure provides a method for continuous, real-time monitoring of concentration levels of an analyte in a biological fluid comprising an extracorporeal blood circuit effluent fluid produced on a hemofilter or a dialyzer device with hemofiltration or dialysis or any combination of these two processes, the method comprising: providing an analyte; providing an analyte receptor and an indicator, wherein at least a portion of the analyte receptor and the indicator form a receptor/indicator complex; contacting the receptor/indicator complex with the analyte; and allowing the analyte to interact with the receptor/indicator complex so as to generate a detectable signal; and detecting the signal by using a Flow Injection Analysis instrument. In some embodiments, the analyte displaces at least a portion of the indicator in the receptor/indicator complex to form a receptor/analyte complex. In other embodiments, the analyte may bind to the receptor/indicator complex.

The present disclosure provides methods that may solve many of the clinical problems associated with CVVH or similar procedures by providing methods that enable the monitoring of analyte concentration levels, such as citrate, calcium, magnesium and phosphate, in real time. For maximum safety, in certain embodiments, the methods may provide a warning of any change in systemic analyte levels so as to prompt the monitoring personnel to review and adjust the treatment settings to ensure the safe continuation of the CVVH or similar procedure. Furthermore, in some embodiments, the methods of the present disclosure may provide information for the fine-tuning of dosages, including calcium plus magnesium dosing, and also monitor the metabolic function of the liver through monitoring the rate of citrate metabolism.

Due to the interaction between citrate and free ionized calcium, the goals of the present disclosure may be achieved by providing a method to measure the concentration levels of an analyte, such as citrate and/or ionized calcium (e.g., free and/or total ionized calcium) in a sample, such as a bodily fluid. In one embodiment, a receptor and an indicator may be provided in the filter effluent fluid line during CVVH. This allows for the indirect measurement of the analyte level in the patient's systemic blood.

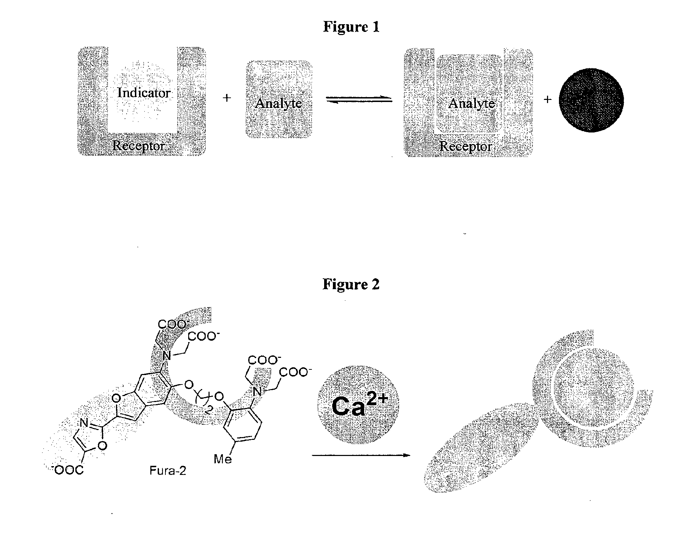

In one embodiment, the methods of the present disclosure may utilize an indicator displacement assay (IDA) for the quantification of an analyte, such as citrate or a different analyte. Figure 1 contains an image depicting an IDA, according to one embodiment of the present disclosure. As shown in Figure 1, IDA is a process in which an analyte receptor is initially allowed to form a weakly associated complex with an indicator, such as a chromophore or fluorophore, and reach an equilibrium. This equilibrium will be affected when an analyte bearing better structural complimentarity to the receptor than the indicator, is introduced into the system. The receptor/indicator complex will start to diminish allowing the receptor/analyte complex to form. At the same time, the indicator in the cavity of the receptor will be released. Due to the variation of the chemical environment of the indicator, its output signal, usually absorption or emission spectra will be modified. This change may be conveniently used in analysis of the analyte concentration provided necessary parameters describing the related equilibria are known.

In another embodiment, the present disclosure provides for the detection of an analyte by allowing the analyte to bind to a receptor/indicator complex. After analyte binding, a detectable signal is produced. One example is shown in Figure 2, which contains an image depicting the binding of ionized calcium to the receptor/indicator complex, Fura-2.

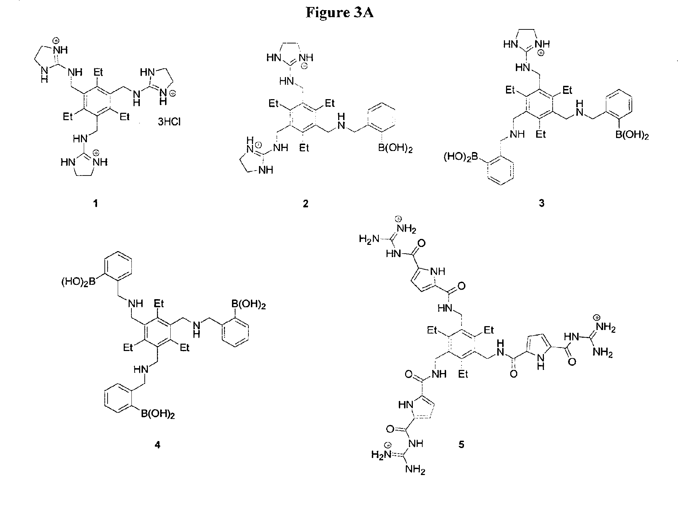

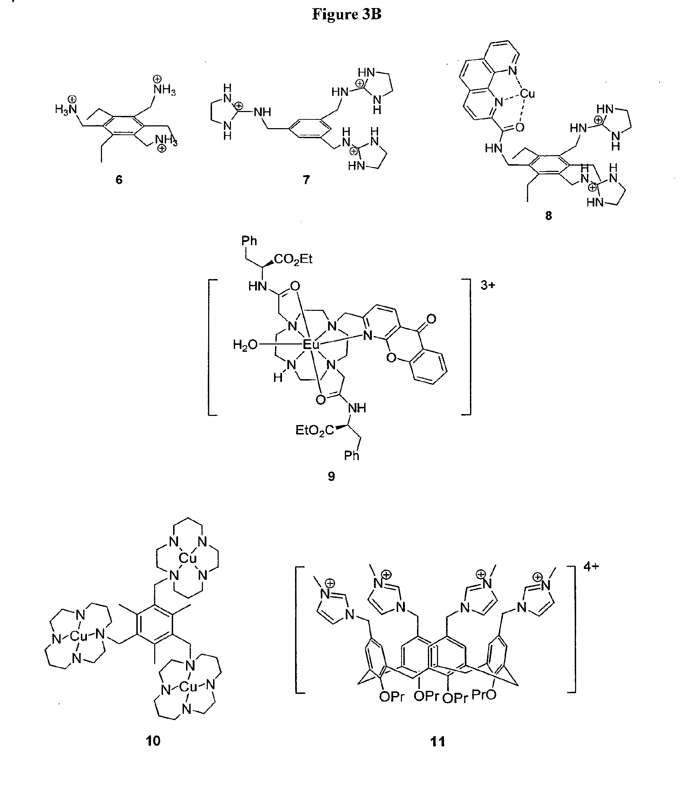

The success of the methods of the present disclosure depend, at least in part, upon the affinity of the receptor or the receptor/indicator complex to bind to the analyte. A variety of different receptors may be used. In certain embodiments, where the analyte is citrate, the receptor is based upon a 2,4,6-triethylbenzene core. However, the receptor can use any scaffold that brings together the functional groups. Various functional groups, including but not limited to guanidinium and phenylboronic acids, are substituted in the 1, 3, and 5 positions. Guanidinium is a favorable functional group because its geometry is conducive for the binding of carboxylates present in citrate and it remains protonated over a wide range pH range. Phenylboronic acid can form robust boronate ester with the α-hydroxy carboxylate moiety of citrate via covalent bonds and represents another favorable functional group for citrate binding. Figures 3A and 3B illustrate several representative citrate receptors. Each of these citrate receptors can be easily synthesized by one of skill in the art. Initial trials have shown that Receptor 2 may be a preferred receptor for citrate. The interactions between the citrate receptor and glucose, fructose, or lactate are insignificant enough to be neglected. Other compounds or ions such as bicarbonate, chloride, phosphate and β-hydroxybutyrate are also expected to cause no interferences.

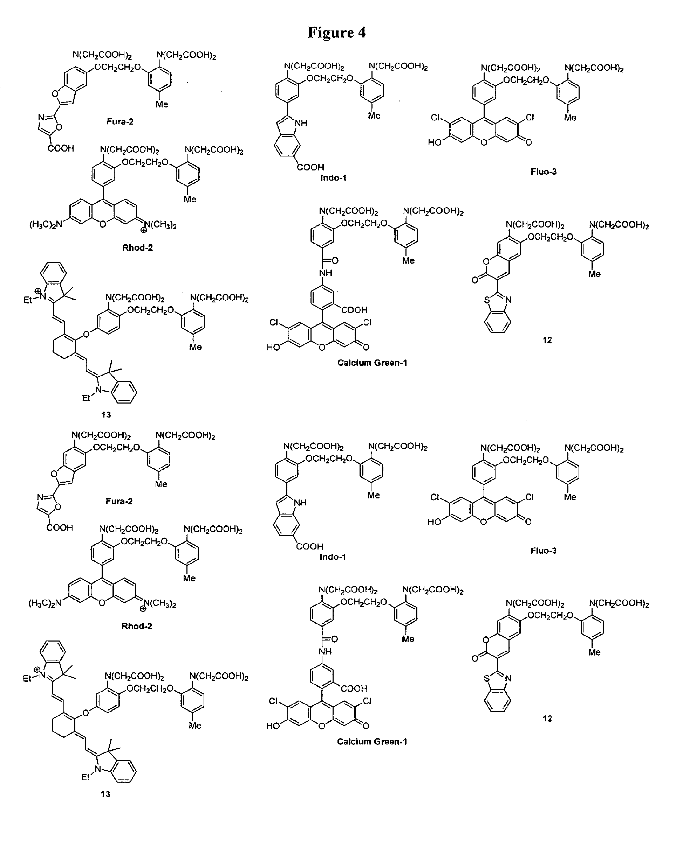

In those embodiments where the analyte is calcium, a variety of Ca²⁺ receptors (only some of which are shown in Figure 4) may be used and are now commercially available from different vendors. Many of them have the common EDTA-mimicking moiety, which forms a stable complex with Ca²⁺ in solution. When such a moiety is appended to a chromophore or fluorophore, modified spectroscopic properties occur after complexation. In one embodiment, the calcium receptor may be Fura-2, which is commercially available from Invitrogen. Owing to its high complexation constant with Ca²⁺, Fura-2 could extract the Ca²⁺ from the complexes with competing anions, such as citrate³⁻, PO₄³⁻, etc. Additionally, Fura-2 shows high selectivity toward Ca²⁺ over other ions such as Mg²⁺, Na⁺, K⁺, etc. The absorption band of Fura-2 is centered at 273 nm. This allows for the detection of Ca²⁺ to take place essentially free from interferences caused by residual proteins in the dialysis fluid, which produce absorption generally below 330 nm.

In those embodiments where the analyte is magnesium, a variety of Mg²⁺ receptors may be used. As would be recognized by one of skill in the art, most current commercially available Mg²⁺ receptors show higher affinity towards Ca²⁺. Therefore, when choosing an appropriate Mg²⁺ receptor, receptors that show an affinity to Mg²⁺ over Ca²⁺ may be selected.

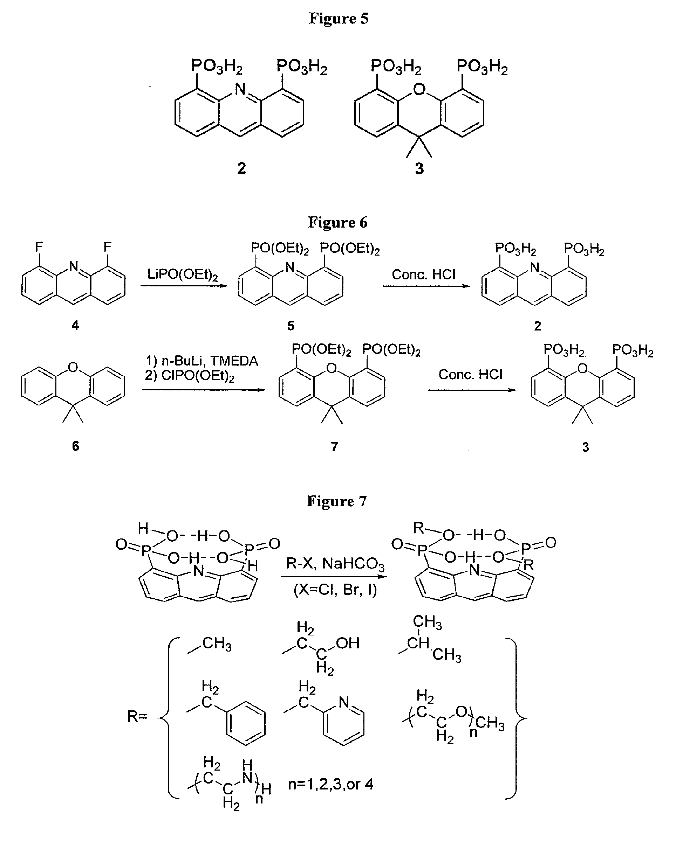

In one embodiment, a suitable Mg²⁺ receptor may include those receptors shown in Figure 5. Receptors 2 and 3 (from Figure 5) may be synthesized from the corresponding acridine or xanthene precursors, as shown in Figure 6. The two fluorine atoms of 4,5-difluoroacridine (4) may be displaced via SN_{AR} mechanism when treated with an appropriate nucleophile. It was reported that negatively charged phophorous species displace fluorine atoms while neutral phosphine does conjugate addition at C-9. Double ortho-lithiation of 9,9-dimethylxanthene (6) is effected by refluxing with n-BuLi and TMEDA in pentane for 10 mins. Quenching with chlorodiehtylphosphate furnishes the ethylphosphonate intermediates. Both 5 and 7 may be easily hydrolyzied by refluxing in concentrated HCl solution to yield the desired receptor 2 and 3, respectively. In addition, the steric and electronic properties of receptors 2 or 3 (from Figure 5) may be further fine tuned via alkylation of the phosphonate group as shown in Figure 7.

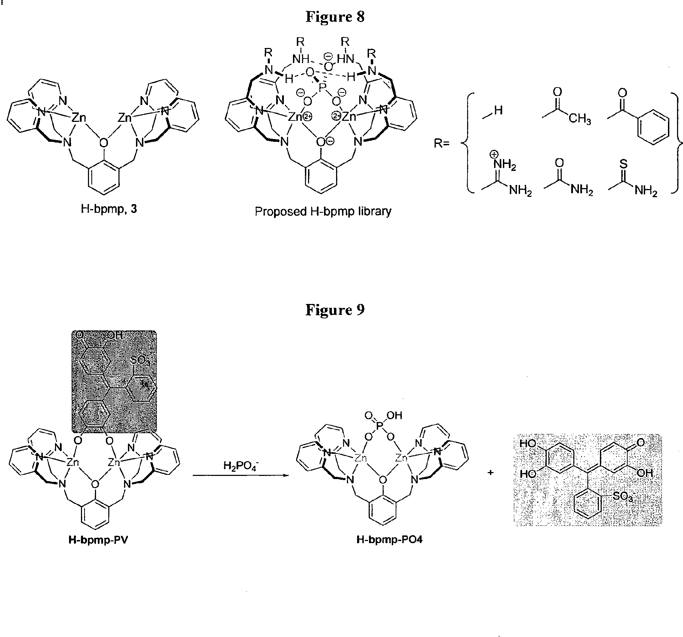

The present disclosure also allows for the testing of phosphate. A number of phosphate receptors with various degrees of selectivity are known in the art. In one embodiment, a suitable phosphate receptor may include those receptors shown in Figure 8. A preferred embodiment uses H-bpmp as reported in (Han, M. S. et. al. Angew. Chem., Int. Ed. 2002, 41, 3809-3811) because it is reported to display selectivity over common anions, such as chloride, bicarbonates, nitrates, etc. The receptor may be synthesized by following the literature procedures. A sensing mechanism according to one embodiments is shown in Figure 9. Pyrocatechol violet (PV) can coordinate to the two Zn²⁺ metal centers in the absence of phosphate or other competing anions. Upon addition of phosphate solution, PV will get displaced and changes to its protonation states will cause the solution color to change from light navy to brownish yellow.

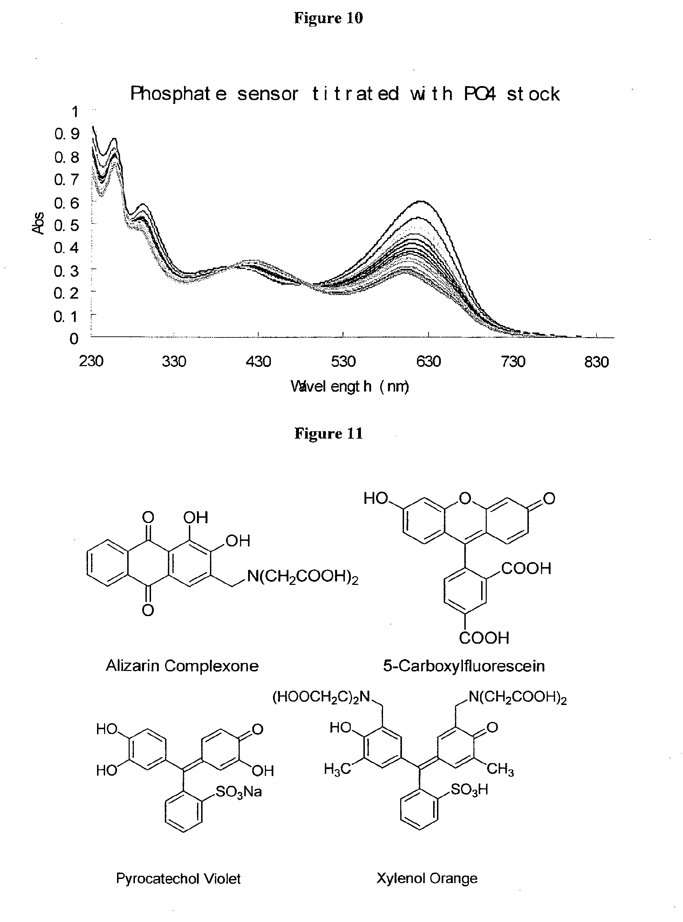

Though phosphate leads to a significant spectral change, as depicted in Figure 10, initial results have shown that citrate can cause severe interferences to the phosphate sensing due to the fact that citrate displays a higher affinity towards the H-bpmp receptor. In such systems where two interfering variants are to be determined, it would be necessary to introduce the use of pattern recognition. When phosphate sensing is performed, the change to the readout signal is not only determined by the concentration of phosphate but also the concentration of citrate in the solution. The same is also true when citrate sensing is performed, though the influence of phosphate is minimal. A mathematical treatment such as artificial neural network (ANN) processing of the raw data can help extrapolate the actual concentration of both phosphate and citrate. In one embodiment, processing of UV-Vis measurements may be accomplished using Statistica Artificial Neural Network software.

The use of a solvent system comprising 75% MeOH: 25% aqueous buffer (v/v) instead of 100% aqueous buffer solution is found to improve selectivity toward phosphate over citrate. This may lead to higher accuracy in phosphate measurements. Additionally, the stability of the phosphate sensing ensemble solution is also dramatically improved in such solvent system.

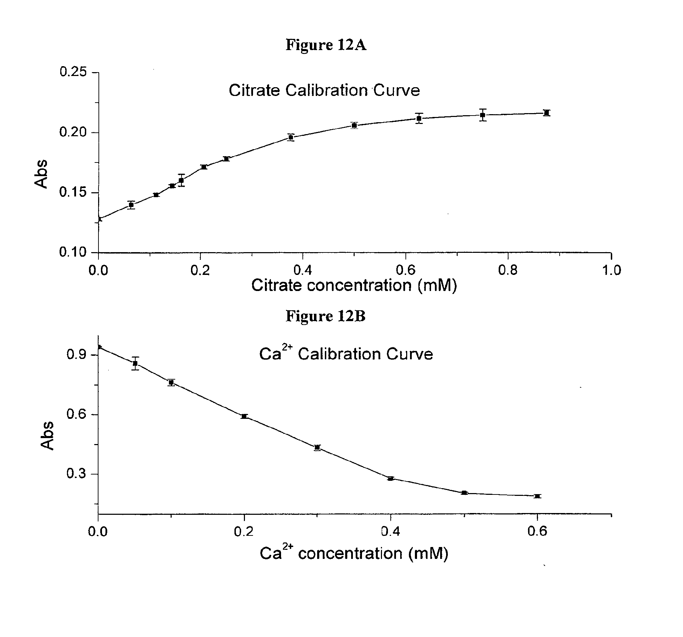

Indicators that are suitable for use in the present disclose include those indicators that are capable of producing a detectable signal when displaced from a receptor/indicator complex by an analyte or those that are capable of producing a detectable signal when an analyte is bound to the receptor/indicator complex. Examples of suitable indicators include, but are not limited to, a chromophore, a fluorophore, alizarin complexone, 5-carboxyfluorescein, pyrocatechol violet, and xylenol orange. Figure 11 illustrates some representative indicators, which are featured with either a catechol moiety or multiple anionic residues. In one embodiment, alizarin complexone is used as the indicator for analysis of citrate concentrations. Alizarin complexone displays a relatively high binding affinity with Receptor 2 originating from the reversible boronic acid/diol interaction. This interaction between receptor and indicator is strong enough to allow the receptor/indicator complex to form to a great extent thus a large spectral change is attained. This is particularly advantageous in minimizing the errors when performing the citrate concentrate measurement activities. However, the strength of the association is still moderate enough to allow the indicator to be displaced by citrate to essentially completion. A calibration curve may be created by plotting the absorbance of a particular wavelength of light at known concentrations of citrate or another analyte. Figures 12A and 12B show representative calibration curves for citrate and calcium. Later, the concentration of an unknown sample may be determined simply by checking the UV-Vis absorption and comparing to the established calibration curve. It is important to account for temperature, however, as temperature affects the equilibrium significantly. Changes of room temperature during the analysis may lead to biased results.

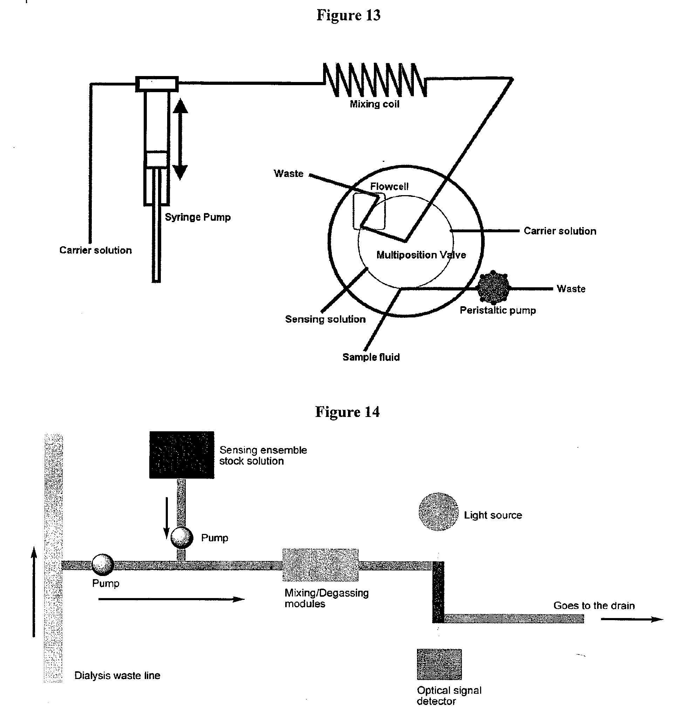

After a detectable signal has been generated, the signal is detected through the use of a Flow Injection Analysis (FIA) instrument. For example, a Sequential Injection Analysis (SIA) System from Ocean Optics, Inc. may be used. In some embodiments, this method of detection may be particularly advantageous as a general UV/vis spectrophotomer is quite space demanding. The SIA System has dimensions of 5"x6"x6" and weighs about 8 lbs. It can also automate liquid transferring and mixing with precise control of volumes with the aid of a personal computer. A build-in compact UV-Vis photometer can then acquire the absorption spectra and the obtained data can be simultaneously analyzed. The working principle of this SIA instrument is shown in Figure 13. An aliquot of sensing solution and dialysis fluid is aspirated into the mixing coil before further pushed into the built-in flowcell for optical signal measurement. This SIA device allows intermittent measurements to be done in an automatic fashion. The frequency can be as fast as 1 minute or so depending on the programs for a specific application.

In another embodiment, an instrument based on the FIA working principle depicted in Figure 14 may be used to measure in a continuous, real-time fashion. In one embodiment, dialysis fluid to be tested is pumped into a line leading to the Flow-Injection-Analysis (FIA) instrument at a steady speed. An aliquot of sensing ensemble stock solution for a certain analyte, e.g., Ca²⁺, citrate, magnesium, phosphate, etc., is pumped into the line to get mixed with the dialysis fluid and induce an optical signal, which is collected by various detectors, e.g., UV-Vis spectrophotometer, CCD cameras, etc. Though optical signals are generally preferred, other methods may be considered as well if they are advantageous in certain circumstances, including but not limited to near-infrared spectroscopy, Raman spectroscopy, Potentiometry, etc). A degassing module could be of use in case gas bubbles are generated during mixing.

To facilitate a better understanding of the present disclosure, the following examples of certain aspects of some embodiments are given. In no way should the following examples be read to limit, or define, the entire scope of the disclosure.

### EXAMPLE 1

An aqueous solution containing all the essential components of a typical dialysis fluid, except for citrate, Ca²⁺, Mg²⁺ and CO₂ is prepared. A 100 mM HEPES buffer with pH at 7.40 is prepared from the above stock solution. The citrate sensing ensemble is prepared as following: 1) mixing 75 mL of MeOH and 25 mL HEPES buffer, 2) dissolving the particular amount of citrate Receptor 2 and alizarin complexone to make their concentrations 100 µM and 250 µM respectively.

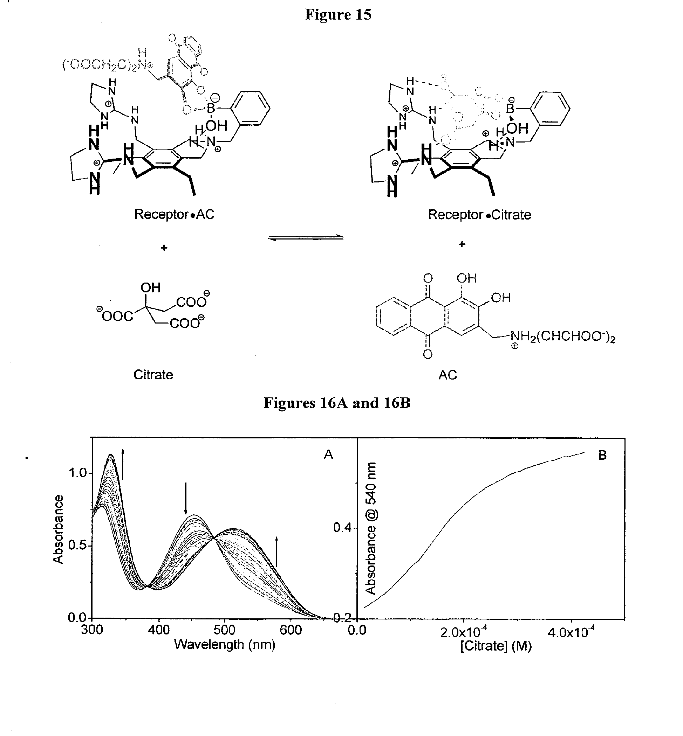

Upon the addition of citrate into the sensing ensemble, alizarin complexone is displaced from the cavity of Receptor 2, yielding to the larger affinity constant between Receptor 2 and citrate. Besides the boronic acid/diol interaction, charge pairing provides an extra driving force for the complexation between the citrate and the Receptor 2 (Figure 15).

Figure 16A demonstrates the change in the absorption spectra of Alizarin complexone when in and outside of the receptor cavity. As the citrate concentration increases, absorption maxima of alizarin complexone at 337 nm and 540 nm increase while the maximum at 447 nm decreases. A calibration curve is made by plotting the solution absorbance at 540 nm vs. the corresponding citrate concentration (Figure 16B).

### EXAMPLE 2

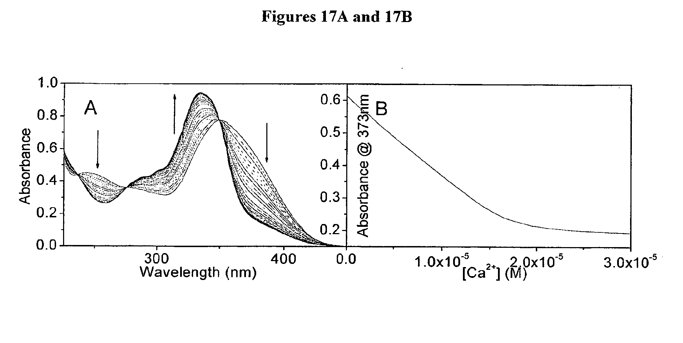

A solution of Fura-2 at 25 µM is prepared using the stock solution mentioned above. An aliquot of sample containing Ca²⁺ is added and changes in the UV-Vis spectrum are observed. As the Ca²⁺ concentration increases, the absorption maxima at 373 nm decreases while the maximum at 330 nm increases (Figure 17A). A calibration curve is made by plotting the solution absorbance at 373 nm vs. the corresponding Ca²⁺ concentration (Figure 17B). The Ca²⁺ concentration of an unknown sample may be obtained by its addition into the Fura-2 solution, checking the absorbance at 373 nm and comparing to the calibration curve. Fura-2 displays such a high binding constant with Ca²⁺ that: 1) Mg²⁺, another prevalent divalent cation present in the dialysate fluid, doesn't interfere, 2) citrate, which has a relatively weak binding affinity to Ca²⁺, doesn't displace Fura-2 in Ca²⁺ binding.

### EXAMPLE 3

Eight patient dialysate fluid sample obtained from ICU unit of the Henry Ford Hospital was tested for [Ca²⁺] and [Citrate] using the calibration curves shown in Figures 12A and 12B. The Ca²⁺ and citrate concentrations in the dialysate fluid were calculated based on the absorption spectra of the resulting solutions (Table 1).

**TABLE 1**

| | Sample ID | ICU-1 | ICU-2 | ICU-3 | ICU-4 | ICU-5 | ICU-6 | ICU-7 | ICU-8 |
|---|---|---|---|---|---|---|---|---|---|
| Ca²⁺ data | Abs | 0.8895 | 0.9162 | 0.9369 | 0.8986 | 0.8232 | 0.7823 | 0.7511 | 0.7013 |
| | Stdev | 0.0278 | 0.0281 | 0.0301 | 0.0270 | 0.0278 | 0.0236 | 0.0222 | 0.0249 |
| | VC | 0.031 | 0.031 | 0.032 | 0.030 | 0.034 | 0.030 | 0.030 | 0.035 |
| | Conc (mM) | 0.063 | 0.030 | 0.004 | 0.052 | 0.137 | 0.180 | 0.214 | 0.272 |
| Citrate data | Abs | 0.1682 | 0.1682 | 0.1661 | 0.1650 | 0.1850 | 0.1872 | 0.1845 | 0.1720 |
| | Stdev | 0.0028 | 0.0026 | 0.0022 | 0.0049 | 0.0031 | 0.0032 | 0.0044 | 0.0013 |
| | VC | 0.017 | 0.015 | 0.013 | 0.030 | 0.017 | 0.017 | 0.024 | 0.008 |
| | Conc (mM) | 3.10 | 3.10 | 2.96 | 2.89 | 4.75 | 5.00 | 4.70 | 3.36 |
| Notes: | | | | | | | | | |
| Abs: average absorbance from multiple replicates. | | | | | | | | | |
| Stdev: standard deviation of the absorbance data from multiple replicates. | | | | | | | | | |
| VC: coefficient of variation calculated by Stdev/Abs. | | | | | | | | | |
| Conc: the concentration of the analyte of the interest in the original ICU samples in the unit of millimolar. | | | | | | | | | |
| ICU samples are diluted with equal amount of 10mM HEPES buffer at pH 7.4 prior to the Ca²⁺ measurements. | | | | | | | | | |
| ICU samples are diluted with 3 volumes of 100mM HEPES buffer at pH 7.4 and 12 volume of MeOH prior to the citrate measurements. | | | | | | | | | |

## Claims

1. A method for continuous, real-time monitoring of concentration levels of an analyte in a biological fluid comprising an extracorporeal blood circuit effluent fluid produced on a hemofilter or a dialyzer device with hemofiltration or dialysis or any combination of these two processes, the method comprising continuously:
providing a biological fluid comprising an analyte;
providing an analyte receptor and an indicator, wherein at least a portion of the analyte receptor and the indicator form a receptor/indicator complex;
contacting the receptor/indicator complex with the analyte;
allowing the analyte to interact with the receptor/indicator complex so as to generate a detectable signal; and
detecting the signal by using a Flow Injection Analysis instrument.

2. A system for continuous, real-time monitoring of concentration levels of an analyte in a biological fluid comprising an extracorporeal blood circuit effluent fluid produced on a hemofilter or a dialyzer device with hemofiltration or dialysis or any combination of these two processes, the system comprising:
a receptor/indicator complex comprising an analyte receptor and an indicator; and an analyte, wherein the analyte will displace the indicator in the receptor/indicator complex; and wherein the displaced indicator will generate a detectable signal; and
a Flow Injection Analysis instrument.

3. The method of Claim 1, or the system of Claim 2, wherein the biological fluid comprises an extracorporeal blood circuit effluent fluid produced on a hemofilter or a dialyzer device with hemofiltration or dialysis or any combination of these two processes.

4. The method of Claims 1 or 3, wherein the concentration level of the analyte in the biological fluid is monitored.

5. The method of Claims 1, 3 or 4, further comprising correlating the detectable signal with a calibration curve to determine a concentration of the analyte.

6. The method of Claim 1 or system of Claim 2, wherein the analyte is selected from the group consisting of: ionized calcium, citrate, phosphate and magnesium.

7. The method of Claim 1, or system of Claim 2, wherein allowing the analyte to interact with the receptor/indicator complex comprises allowing the analyte to displace at least a portion of the indicator in the receptor/indicator complex to form a receptor/analyte complex.

8. The method of Claim 1, or system of Claim 2, wherein allowing the analyte to interact with the receptor/indicator complex comprises allowing the analyte to bind to the receptor/indicator complex.

9. The method of Claim 1, or system of Claim 2, wherein the indicator comprises at least one indicator selected from the group consisting of: a chromophore, a fluorophore, alizarin complexone, 5-carboxyfluorescein, pyrocatechol violet, and xylenol orange.

10. The method of Claim 1, or system of Claim 2, wherein the analyte comprises phosphate and citrate.

11. The method of Claim 10, further comprising using a mathematical treatment to extrapolate the concentration of phosphate and citrate.

12. The method of Claim 11, wherein the mathematical treatment comprises an artificial neural network (ANN).

## Patentansprüche

1. Verfahren zur kontinuierlichen Echtzeitüberwachung von Konzentrationsspiegeln eines Analyts in einem biologischen Fluid, das ein Ablauffluid eines extrakorporalen Blutkreislaufs umfasst, das an einem Hämofilter oder einer Dialysevorrichtung mit Häfiltration oder Dialyse oder einer Kombination von diesen beiden Verfahren produziert wurde, wobei das Verfahren kontinuierlich umfasst:
Bereitstellen eines biologischen Fluids, das einen Analyt umfasst;
Bereitstellen eines Analytrezeptors und eines Indikators, wobei mindestens ein Teil des Analytrezeptors und des Indikators einen Rezeptor/Indikator-Komplex bilden;
Inkontaktbringen des Rezeptor/Indikator-Komplexes mit dem Analyt;
Ermöglichen einer Wechselwirkung des Analyts mit dem Rezeptor/Indikator-Komplex derart, dass ein detektierbares Signal erzeugt wird; und
Detektieren des Signals unter Verwendung eines Fließinjektionsanalyse-Instruments.

2. System zur kontinuierlichen Echtzeitüberwachung von Konzentrationsspiegeln eines Analyts in einem biologischen Fluid, das ein Ablauffluid eines extrakorporalen Blutkreislaufs umfasst, das an einem Hämofilter oder einer Dialysevorrichtung mit Hämofiltration oder Dialyse oder einer Kombination von diesen beiden Verfahren produziert wurde, wobei das System umfasst:
einen Rezeptor/Indikator-Komplex, der einen Analytrezeptor und einen Indikator umfasst; und
einen Analyt, wobei der Analyt den Indikator in dem Rezeptor/Indikator-Komplex verdrängt; und wobei der verdrängte Indikator ein detektierbares Signal erzeugt; und
ein Fließinjektionsanalyse-Gerät.

3. Verfahren nach Anspruch 1 oder System nach Anspruch 2, wobei das biologische Fluid ein Ablauffluid eines extrakorporalen Blutkreislaufs, das an einem Hämofilter oder einer Dialysevorrichtung mit Hämofiltration oder Dialyse oder einer Kombination von diesen beiden Verfahren produziert wurde, umfasst.

4. Verfahren nach Anspruch 1 oder 3, wobei der Konzentrationsspiegel des Analyts in dem biologischen Fluid überwacht wird.

5. Verfahren nach Anspruch 1, 3 oder 4, das ferner eine Korrelation des detektierbaren Signals mit einer Eichkurve zum Bestimmen einer Konzentration des Analyts umfasst.

6. Verfahren nach Anspruch 1 oder System nach Anspruch 2, wobei der Analyt aus der Gruppe von ionisiertem Calcium, Citrat, Phosphat und Magnesium ausgewählt ist.

7. Verfahren nach Anspruch 1 oder System nach Anspruch 2, wobei das Ermöglichen einer Wechselwirkung des Analyts mit dem Rezeptor/Indikator-Komplex das Ermöglichen des Verdrängens von mindestens einem Teil des Indikators in dem Rezeptor/Indikator-Komplex durch den Analyt unter Bildung eines Rezeptor/Analyt-Komplexes umfasst.

8. Verfahren nach Anspruch 1 oder System nach Anspruch 2, wobei das Ermöglichen einer Wechselwirkung des Analyts mit dem Rezeptor/Indikator-Komplex das Ermöglichen einer Bindung des Analyts an den Rezeptor/Indikator-Komplex umfasst.

9. Verfahren nach Anspruch 1 oder System nach Anspruch 2, wobei der Indikator mindestens einen Indikator umfasst, der aus der Gruppe von einem Chromophor, einem Fluorophor, Alizarin-Komplexon, 5-Carboxyfluorescein, Brenzcatechinviolett und Xylenolorange ausgewählt ist.

10. Verfahren nach Anspruch 1 oder System nach Anspruch 2, wobei der Analyt Phosphat und Citrat umfasst.

11. Verfahren nach Anspruch 10, das ferner die Verwendung einer mathematischen Behandlung zur Extrapolation der Konzentration von Phosphat und Citrat umfasst.

12. Verfahren nach Anspruch 11, wobei die mathematische Behandlung ein künstliches neuronales Netz (KNN) umfasst.

## Revendications

1. Procédé de surveillance continue en temps réel de niveaux de concentration en un analyte dans un fluide biologique, comprenant un fluide effluent d'un circuit sanguin extracorporel produit sur un hémofiltre ou un dispositif dialyseur avec hémofiltration ou dialyse ou toute combinaison de ces deux processus, le procédé comprenant de façon continue :
la fourniture d'un fluide biologique comprenant un analyte ;
la fourniture d'un récepteur d'analyte et d'un indicateur, dans lequel au moins une partie du récepteur d'analyte et de l'indicateur forme un complexe récepteur/indicateur ;
la mise en contact du complexe récepteur/indicateur avec l'analyte ;
le fait de laisser l'analyte interagir avec le complexe récepteur/indicateur de façon à générer un signal détectable ; et
la détection du signal à l'aide d'un instrument d'analyse par injection de flux.

2. Système de surveillance continue en temps réel de niveaux de concentration en un analyte dans un fluide biologique, comprenant un fluide effluent d'un circuit sanguin extracorporel produit sur un hémofiltre ou un dispositif dialyseur avec hémofiltration ou dialyse ou toute combinaison de ces deux processus, le système comprenant :
un complexe récepteur/indicateur comprenant un récepteur d'analyte et un indicateur ; et un analyte, dans lequel l'analyte va déplacer l'indicateur dans le complexe récepteur/indicateur ; et dans lequel l'indicateur déplacé va générer un signal détectable ; et
un instrument d'analyse par injection de flux.

3. Procédé selon la revendication 1, ou système selon la revendication 2, dans lequel le fluide biologique comprend un fluide effluent d'un circuit sanguin extracorporel produit sur un hémofiltre ou un dispositif dialyseur avec hémofiltration ou dialyse ou toute combinaison de ces deux processus.

4. Procédé selon les revendications 1 ou 3, dans lequel le niveau de concentration en analyte dans le fluide biologique est surveillé.

5. Procédé selon les revendications 1, 3 ou 4, comprenant en outre la corrélation du signal détectable avec une courbe d'étalonnage afin de déterminer une concentration de l'analyte.

6. Procédé selon la revendication 1, ou système selon la revendication 2, dans lequel l'analyte est choisi dans le groupe constitué par : le calcium ionisé, le citrate, le phosphate et le magnésium.

7. Procédé selon la revendication 1, ou système selon la revendication 2, dans lequel le fait de laisser l'analyte interagir avec le complexe récepteur/indicateur comprend le fait de laisser l'analyte déplacer au moins une partie de l'indicateur dans le complexe récepteur/indicateur afin de former un complexe récepteur/analyte.

8. Procédé selon la revendication 1, ou système selon la revendication 2, dans lequel le fait de laisser l'analyte interagir avec le complexe récepteur/indicateur comprend le fait de laisser l'analyte se lier au complexe récepteur/indicateur.

9. Procédé selon la revendication 1, ou système selon la revendication 2, dans lequel l'indicateur comprend au moins un indicateur choisi dans le groupe consistant en : un chromophore, un fluorophore, une complexone d'alizarine, la 5-carboxyfluorescéine, le violet de pyrocatéchol et l'orange de xylénol.

10. Procédé selon la revendication 1, ou système selon la revendication 2, dans lequel l'analyte comprend du phosphate et du citrate.

11. Procédé selon la revendication 10, comprenant en outre l'utilisation d'un traitement mathématique pour extrapoler la concentration en phosphate et citrate.

12. Procédé selon la revendication 11, dans lequel le traitement mathématique comprend un réseau de neurones artificiels (NRA).
